# EUROPEAN PATENT APPLICATION

(11) **EP 1 354 943 A2**
(43) Date of publication of application: **22.10.2003**
(21) Application number: 03251974.6
(22) Date of filing: 28.03.2003
(51) Int. Cl.: C12N 5/22, C12N 5/06, C07K 16/28, G01N 33/577

(54) **Monoclonal antibodies, hybridomas, cell isolation method, isolated cells and immunological diagnostic method**

(30) Priority: 28.03.2002 JP 2002090863
(71) Applicant: National Institute of Advanced Industrial Science and Technology, Tokyo 100-8921 (JP)
(72) Inventor: Hara, Masayuki c/o Nat. Inst. of Adv.Ind. Science, Ikeda-shi, Osaka-fu 563-8577 (JP); Kanemura, Yonehiro c/o Nat. Inst. of Adv.Ind., Ikeda-shi, Osaka-fu 563-8577 (JP); Miyake, Jun c/o Nat. Inst. of Adv.Ind., Ikeda-shi, Osaka-fu 563-8577 (JP); Okano, Hideyuki c/o Nat. Inst. of Adv.Ind., Ikeda-shi, Osaka-fu 563-8577 (JP); Yamasaki, Mami c/o Nat. Inst. of Adv.Ind., Ikeda-shi, Osaka-fu 563-8577 (JP); Nakamura, Yasuhiro c/o Nat. Inst. of Adv.Ind., Ikeda-shi, Osaka-fu 563-8577 (JP); Yamamoto, Munehiko c/o Nat. Inst. of Adv.Ind., Ikeda-shi, Osaka-fu 563-8577 (JP); Oda, Eriko c/o Nat. Inst. of Adv.Ind., Ikeda-shi, Osaka-fu 563-8577 (JP); Kodama, Eri c/o Nat. Inst. of Adv.Ind., Ikeda-shi, Osaka-fu 563-8577 (JP)
(74) Representative: Daniels, Jeffrey Nicholas

(57) **Abstract**

The present invention provides monoclonal antibodies useful in selection/isolation/preparation of a cell derived from a nerve tissue or other tissue, and in the examination of site-specific expression of proteins in brain nerve tissue. The present invention also provides hybridomas which produce the monoclonal antibodies, a method for isolation of a cell derived from a nerve tissue or other tissue using the monoclonal antibodies, a cell isolated through the method, and an immunological diagnostic method using the monoclonal antibodies.

## Description

### FIELD OF THE INVENTION

The present invention relates to monoclonal antibodies useful in selection/isolation/ preparation of a cell derived from a neural tissue or other tissue, and in the examination of site-specific expression of proteins in central nervous tissue. The present invention also relates to hybridomas which produce the monoclonal antibodies, a method for isolation of a cell derived from a nerve tissue or other tissue using the monoclonal antibodies, a cell isolated through the method, and an immunological diagnostic method using the monoclonal antibodies.

### BACKGROUND OF THE INVENTION

Heretofore, medical treatments using organ transplantation and implantation of artificial organs have been developed and implemented to repair damaged or lost human tissues due to intractable diseases or external injuries. Artificial organs are still in a developmental phase, and only few permanently implantable artificial organs are now in use. Organ transplantation has suffered from a fundamental limitation imposed by an insufficient number of donors, and has not been established as a common practice due to rejection and ethical problems.

Under such circumstances, as an alternative to treatment using artificial organs and organ transplantation, research on regeneration treatments has recently become active. The regeneration treatments are based on stem cells having pluripotency or a capability of differentiating into a plurality of precursor cells and differentiated cells that may be used to produce a particular structure. In regeneration treatment, a stem cell having pluripotency is proliferated and differentiated through culturing, and then the differentiated cells are returned to their tissue of origin to achieve a desired tissue regeneration. Regeneration treatment is highly anticipated as a potential alternative to treatment using artificial organs and organ transplantation.

Stem cells can be roughly classified into embryonic stem cells and somatic stem cells. Both types are applicable to regeneration treatment, and related research is being conducted. While embryonic stem cells are regarded as a more noteworthy type, capable of infinitely proliferating and differentiating into any cell type, an ethical issue is raised over their use in humans because research on human embryonic stem cells derived from fertilized human eggs could be used in the creation of designer babies or clones.

Somatic stem cells, which are more differentiated than embryonic stem cells, can be further differentiated into only a limited group of cells. In addition, they evidently exist in adult tissue, and thus there is no need for using fertilized human eggs. Therefore, somatic stem cells are expected to be a safe cell type, meeting the needs of human regeneration treatment.

It has been believed that cell differentiation in the central nervous system, such as in the brain and spinal cord, is completed shortly after birth and no cell regeneration will occur in adults if the central nervous system is damaged by accidents or diseases, such as in Alzheimer's disease and brain hemorrhage. Recently, however, it has been verified that the human adult brain has neural stem cells, and that the stem cells can be proliferated and differentiated under certain conditions. Thus, it is believed that if a technique of adequately controlling the proliferation/differentiation of neural stem cells is established, these cells could be used as an effective treatment for brain nerve disorders.

In order to use somatic stem cells in regeneration treatment, a stem cell or precursor stem cell needs to be differentiated and proliferated to a required number for the treatment. As a prerequisite therefor, it is necessary to efficiently isolate from some source a stem cell capable of differentiating into target cells or tissue.

An isolation method using FACS (Fluorescence-Activated Cell Sorting) is known as one technique for efficiently isolating stem cells. This method uses a fluorescence-labeled monoclonal antibody for recognizing an expression pattern of a surface marker selectively expressed on a stem cell, and isolates the stem cell according to the resulting fluorescence intensity of the monoclonal antibody.

No surface marker for neural stem cells has been reported, with the exception of a surface marker recognized by CD133 antibody. This CD133 antibody can directly recognize a neural stem cell and react therewith. However, the thus isolated neural stem cell inevitably contains deposited fluorescent material from the CD133 antibody. The long-term safety in medical use of cells from proliferation/differentiation of the neural stem cell with the deposited fluorescent material has not been verified yet and is a concern.

### SUMMARY OF THE INVENTION

If an antibody which selectively recognizes a neural stem cell or a neural precursor cell among neural cells is produced, or if, in a reverse way, a monoclonal antibody which selectively recognizes a cell other than the neural stem cell or the neural precursor cell is produced, such antibodies could be used to safely and efficiently isolate stem cells and precursor cells, and such cells could be used in medical treatments. Based on this premise, the inventors continuously conducted research.

During the course of the research, the inventors produced novel monoclonal antibodies by using homogenized cells derived from human neural tissue as a sensitizing antigen.

The inventors then verified that some of the antibodies they had produced recognized an antigen expressed on cells localized in the ependymal and subependymal layers of human brain tissue. They also verified that other antibodies they had produced recognized an antigen expressed on cells existing in a region other than the ependymal and subependymal layers of human brain tissue, and not the antigen expressed on the cells localized in the ependymal and subependymal layers of human brain tissue. The inventors also verified that each of these antibodies could be used in stem cell selection, and thus accomplished the present invention.

Specifically, it is an object of the present invention to provide a novel monoclonal antibody which selectively recognizes and binds a neural stem cell or a neural precursor cell that may be used to efficiently isolate such cells.

It is another object of the present invention to provide a novel monoclonal antibody which recognizes and binds to a cell other than a neural stem cell or a neural precursor cell that may be used to efficiently isolate a neural stem cell or a neural precursor cell which is not recognized and bound by the antibody.

It is still another object of the present invention to provide a hybridoma which produces one of the monoclonal antibodies described above.

It is yet another object of the present invention to provide a method of isolating a neural stem cell or a neural precursor cell using one of the monoclonal antibodies described above.

It is still a further object of the present invention to provide a cell derived from a neural stem cell or a neural precursor cell or from a tissue other than nerve tissue, which is isolated through the method of the present invention.

It is another object of the present invention to provide an immunological diagnostic method using the monoclonal antibodies described above.

In order to achieve the above objects, according to a first aspect of the present invention, there is provided a monoclonal antibody which selectively recognizes an antigen expressed on a neural stem cell or a neural precursor cell localized in the ependymal and subependymal layers of the human brain.

According to a second aspect of the present invention, there is provided a monoclonal antibody that selectively immunostains the ependymal and subependymal layers of the human brain in an immunostaining procedure using human brain tissue slices.

According to a third aspect of the present invention, there is provided a monoclonal antibody which recognizes an antigen expressed on a cell residing in a region other than the ependymal and subependymal layers of the human brain, and does not recognize an antigen expressed on a cell localized in the ependymal and subependymal layers.

According to a fourth aspect of the present invention, there is provided a monoclonal antibody that selectively immunostains a region of the human brain other than the ependymal and subependymal layers in an immunostaining procedure using human brain tissue slices.

According to a fifth aspect of the present invention, there is provided a method of isolating a cell using the monoclonal antibody set forth in any one of the first to fourth aspects of the present invention.

According to a sixth aspect of the present invention, there is provided a cell which is isolated through the method set forth in the fifth aspect of the present invention.

The above monoclonal antibodies of the third aspect of the invention are operative to selectively recognize and bind an antigen expressed on a cell other than a neural stem cell or a neural precursor cell and they may be used to efficiently isolate neural stem cells or neural precursor cells. Thus, use of these monoclonal antibodies can advantageously result in the isolation of a neural stem cell or a neural precursor cell in safety without deposit of fluorescent materials in the isolated neural stem cells or neural precursor cells.

The monoclonal antibodies of the present invention can be prepared as follows.

The monoclonal antibodies of the present invention are conceptually prepared by immunizing an animal using a particular cell through a conventional immunization method, for example, using human fetal brain cells as a sensitizing antigen. Antibody-producing cells are then fused with another cell through a cell fusion method, the fused cell is cloned through a conventional cloning method, and the cloned cells are then screened.

More specifically, in the method of the present invention, the monoclonal antibody may be prepared by immunizing a mammal using, for example, human fetal brain cells as a sensitizing antigen, fusing the mammal plasma cell (immune cell) with a myeloma cell of a mammal such as a mouse, cloning the obtained fuse cell (hybridoma), selecting a clone producing the antibody of the present invention, which recognizes the aforementioned cell type, from the obtained cloned cells, culturing the selected clone, and collecting the target antibody.

In this monoclonal antibody preparation method, the mammal to be immunized by the sensitizing antigen is not limited to a specific type or kind, but any suitable mammal having compatibility with the myeloma cell for use in the cell fusion, preferably, mice, rats or hamsters, may be selected.

The immunization may be performed by administering the above human fetal brain cells to the mammal through a conventional method, for example, by intraperitoneal injection. Preferably, the human fetal brain cell is diluted and suspended by an appropriate amount of PBS or saline, and then administered to the mammal several times every month. The human fetal brain cells are expected to contain cells from the ependymal and subependymal layers of the human brain, as well as cells from other areas of the human brain. Thus, use of human fetal brain cells as the sensitizing antigen will lead to the production of both antibodies that recognize neural stem cells and neural precursor cells localized in the ependymal and subependymal layers, and also antibodies that recognize a cell residing in a region other than the ependymal and subependymal layers of the human brain.

Preferably, spleen cells removed from the mammal after the last administration of the brain cells are used as the immune cells.

Any suitable conventional cell line may be used as the mammal myeloma cell to be fused with the immune cell. For example, suitable mammal myeloma cells include P3 (P3 X 63 Ag 8.653) (*J. Immunol.,* 123, 1548 (1978)), p3-U1 (*Curr. Topics in Micro, and Immunol.,* 81, 1-7 (1978)), NS-1 (*Eur*. *J*. *Immunol.,* 6, 511-519 (1976)), MPC-11 (*Cell*, 8, 405-415 (1976)), Sp2/0-Ag14 *(Nature,* 276, 269-270 (1978)), FO (*J*. *Immunol. Meth.,* 35, 1-21 (1980)), S194 (*J*. *Exp. Med.,* 148, 313-323 (1978)), and R210 (*Nature,* 277, 131-133 (1979)).

The cell fusion between the immune cells and the myeloma cells may be performed through a conventional method, for example, the method disclosed in (Milstein et al. *Methods Enzymol.,* 73, 3-46 (1981)).

Preferably, the cell fusion is prepared in a conventional nutrient medium in the presence of a fusion accelerator. The fusion accelerator may be polyethylene glycol (PEG), or Hemagglutinating virus (HVJ), and an appropriate amount of auxiliary agent, such as dimethyl sulfoxide, may be added thereto to provide enhance fusion efficiency, according to need. Preferably, the ratio of the immune cell to the myeloma cell is 1:10. The medium for use in the cell fusion may be RPMI-1640 medium or MEM medium suitable for growing the myeloma cell strain. Alternatively, any other suitable medium generally used for culturing this type of cell may also be used as the medium, and serum replenisher, such as fetal bovine serum (FBS), may be used therewith.

The cell fusion may be performed by well mixing the immune cells and the myeloma cells in a given ratio in the medium, then adding a PEG solution preheated to about 37°C to the mixture, followed by additional mixing. The PEG solution contains PEG having, for example, an average molecular mass of 1,000-6,000 daltons, and typically in a concentration of about 30-60 % (W/V). Then, the mixture is incubated for 90 min in a CO₂ incubator at 37°C, then 1 ml of RPMI 1640 without FCS is added and mixture shook, another 1 ml of the same medium is added and the mixture again shook, then 8 ml of the same medium is added. The supernatant of the mixture is removed through centrifugation to collect the fused cells.

The hybridoma may then be selected from the fused cells by culturing the cells in a conventional selective medium such as HAT medium (containing hypoxhanthine, aminopterin and thymidine). The culturing in the HAT medium is continued for a sufficient time required for the death of the unfused cells, typically for several days to several weeks. Then, through a conventional limiting dilution method, the fused cells in the medium are screened to select hybridomas which produce the target antibody, and then a selected hybridoma is cloned to increase its monoclonality.

As above, the hybridoma which produces the monoclonal antibody of the present invention can be sub-cultured in conventional mediums. Further, the hybridoma can be preserved in liquid nitrogen for a long period.

The monoclonal antibodies of the present invention may be appropriately cultivated from the prepared hybridomas through a conventional method of culturing the hybridomas, and collecting the target antibodies from the supernatant of the cultured solutions. Alternatively, the monoclonal antibodies may be cultivated by administering the hybridomas to mammals having compatibility therewith, proliferating the hybridomas in the mammals, and collecting the target antibodies from the ascites of the mammals. The former method is suitable for obtaining the antibodies with high purity. The latter method is suitable for preparing the antibody in a large scale.

The antibodies obtained through the above methods may be purified through a conventional purification technique, such as salting-out, gel filtration or affinity chromatography so as to provide an increased purity.

In one aspect, the monoclonal antibody of the present invention prepared through the above methods can selectively recognize and bind a cell other than a neural stem cell or a neural precursor cell among neural cells, and be used to efficiently isolate the neural stem cells from the human brain tissue. By selectively recognizing and binding cells other than neural stem cells, use of the monoclonal antibody of the present invention can prevent fluorescent material from depositing on the stem cells, thereby ensuring the safety of the isolated stem cell.

In contrast, in the method of isolating a neural stem cell or a neural precursor cell, as set forth in the fifth aspect of the present invention, the above monoclonal antibody recognizes and binds an antigen on the surface of a neural cells, and marks the surface of the cell for use in isolation of the cell.

The isolation of the cells (whether neural stem cells, neural precursor cells, or another cell type) may be achieved through any suitable method of isolating a cell through surface marking using the antibody. The isolation method may include, but is not limited to, a fluorescence-activated cell sorting (FACS) method, a magnetic separation method (using an antibody conjugated to a magnetic bead), and a method in which the antibody has affinity to a protein, so called "cell-specific surface marker."

The method described above, using antibodies that recognize and bind cells other than neural stem cells and neural precursor cells, allows the neural stem cell or the neural precursor cell to be efficiently isolated without contacting them with a fluorescent material that could be incorporated into the cell. The neural stem cells and the neural precursor cells thus obtained can be applied to medical diagnosis or used in regeneration treatments for disorders of the central nervous system, after controlled proliferation and/or differentiation of the cells, without worry as to the effects of the fluorescent material.

The immunological diagnostic method using the monoclonal antibodies of the fifth aspect of the present invention utilizes an antigen-antibody reaction caused by the monoclonal antibody.

The immunological diagnostic method may be applied to various types of immunological or biochemical diagnosis techniques utilizing the antigen-antibody reaction caused by the monoclonal antibody.

For example, the diagnosis techniques include (1) use of a fluorescent antibody or a chemical staining method in which the monoclonal antibody is labeled with dye, such as a fluorescent dye, to allow the presence of the linkage between the monoclonal antibody and the antigen to be visibly observed, (2) an enzyme-antibody method using an enzyme instead of the fluorescent dye to label the monoclonal antibody, (3) an ELISA method using a protein-labeled secondary antibody to measure an amount of antigen or the like, (4) a radioimmunoassay method in which the monoclonal antibody is labeled with isotope, and (5) an immunoprecipitation method utilizing an agglutination reaction caused by the antigen-antibody reaction.

The diagnosis techniques further include a Western blotting method in which proteins are fractionated through electrophoresis, and detected by the monoclonal antibody. The Western blotting method is advantageous in the direct cloning of a polynucleotide encoding the antigen protein with respect to the monoclonal antibody. The Western blotting method includes various modifications, such as a Western method, a South Western method, a North Western method, and a West Western method.

### BRIEF DESCRIPTION OF THE DRAWINGS

Fig. 1 shows the results of Western blotting using the HFB115 antibody.

Fig. 2 is a nuclear stain image of human fetal brain tissue stained by the HFB27 antibody with DNA staining dye TO-PRO-3®.

Fig. 3 is an immunostain image of human fetal brain tissue with Nestin stained by the HFB27 antibody.

Fig. 4 is an immunostain image of human fetal brain tissue stained by the HFB27 antibody.

Fig. 5 is a superimposed multiple stain image of the above three stain images stained by the HFB27 antibody.

Fig. 6 is a nuclear stain image of human fetal brain tissue stained by the HFB16 antibody with DNA staining dye TO-PRO-3®.

Fig. 7 is an immunostain image of human fetal brain tissue with Nestin stained by the HFB 16 antibody.

Fig. 8 is an immunostain image of human fetal brain tissue stained by the HFB16 antibody.

Fig. 9 is a superimposed multiple stain image of the above three stain images stained by the HFB 16 antibody.

Fig. 10 is a nuclear stain image of human fetal brain tissue stained by the HFB115 antibody with DNA staining dye TO-PRO-3®.

Fig. 11 is an immunostain image of human fetal brain tissue with Nestin stained by the HFB 115 antibody.

Fig. 12 is an immunostain image of human fetal brain tissue stained by the HFB115 antibody.

Fig. 13 is a superimposed multiple stain image of the above three stain images stained by the HFB 115 antibody.

Fig. 14 is a nuclear stain image of human fetal brain tissue stained by the HFB211 antibody with DNA staining dye TO-PRO-3®.

Fig. 15 is an immunostain image of human fetal brain tissue with Nestin stained by the HFB211 antibody.

Fig. 16 is an immunostain image of human fetal brain tissue stained by the HFB211 antibody.

Fig. 17 is a superimposed multiple stain image of the above three stain images stained by the HFB211 antibody.

Fig. 18 shows the sorting results of neurosphere cells using the HFB 115 antibody.

Fig. 19 shows the sorting results of the human Jurkat lymphoid cell line using the HFB211 antibody.

Fig. 20A shows the sequence of peptide fragments of the fractionate protein isolated using the HFB 115 antibody.

Fig. 20B shows an alignment between the amino acid sequence of the H1D histone protein and a peptide fragment of the protein isolated using the HFB115 antibody.

Fig. 21 shows a multiple stain image of neurospheres stained with the HFB115 antibody, an anti-Histone H1 monoclonal antibody and an anti-Histone H1 polyclonal antibody.

### DESCRIPTION OF THE PREFERRED EMBODIMENT

Examples of the present invention will now be described in detail.

### 1. METHOD OF PREPARING MONOCLONAL ANTIBODY

### Immunization of Animals:

The monoclonal antibodies of the present invention were prepared according to a modification of a method by Orlik, O & Altaner, C (*J*. *Immunol. Methods.* 115: 55-59, 1998) using inguinal lymph node cells. Homogenates, which were suspensions prepared by mashing human brain tissue of 9, 14 and 23 weeks of fetal age, were used as an antigen. The antigen was injected into 4-5 female Balb/c mice (6 weeks old) in an amount of 0.2 ml through both of their plantas (bottom of the feet), three times at 3-4 days intervals, in the form of a mixture with RIBI Adjuvant (Funakoshi) each time. After 5 days from the completion of these administrations, the antigen was injected without adjuvant as the last booster.

### Preparation of Hybridoma Cells:

FO-1 cells, an established myeloma cell line, were cultured in RPMI 1640 medium (GIBCO, Life Technology) containing 15% fetal calf serum (FCS) and 8-azaguanine (20 µg/ml), at 37°C using a CO₂ incubator for 3 days. In advance of the cell fusion process, 8-azaguanine was removed from the culture medium, and the cell culture was continued for 2-3 days.

After 3 days from the last booster, the cell fusion process was performed as follows to prepare hybridoma cells.

The bilateral inguinal lymph nodes of 4-5 mice were removed. The removed lymph nodes were put in a RPMI 1640 media without FCS, and ground while sandwiching between two ground-glasses to release the lymphocytes, resulting in about 1 x 10⁸ cells. Then, an aliquot of 2-4 x 10⁷ FO-1 cells was prepared.

The cells from the first and second processes were mixed in 50 ml of proliferation medium (RPMI 1640 + 15% FCS). The mixture was centrifuged at 1000 rpm (revolutions per minute) for 5 minutes at room temperature, and then the supernatant was discarded. The cells were suspended in 20 ml of the proliferation medium.

10 µl of a 50% polyethylene glycol (PEG) solution was added to the suspension, and the mixture was then kept at 37°C in a CO₂ incubator for 90 minutes. After centrifugation at 1000 rpm for 5 minutes, the supernatant was discarded, and the cells were again centrifuged for several seconds in order to remove as much of the supernatant as possible.

A vessel containing the collected cells was slightly hit against the water surface of a constant temperature water bath of 37°C to warm and loose the centrifuged deposit in the sample.

1 ml of a 50% polyethylene glycol (PEG) solution preheated at 37°C was slowly added to the cells over 1 minute, and the mixture was shaken. Then, an additional 1 ml of the PEG solution was slowly added to the cells over 1 minute, and the mixture was again shaken. Then, 8 ml of the PEG solution was added to the cells over 2-3 minutes. The mixture was stirred, then centrifuged at 1000 rpm, and the supernatant discarded.

The centrifuged deposit consisting of the cells was resuspended in 50 ml of HAT medium (2 ml of 50 x HAT (Sigma Corp.) per 100 ml proliferation medium), and seeded on and cultured in 96 well plates (5 plates) at 0.1 ml/well. During this process, thymus cells were added to each of the plates at about 1 x 10⁵ cells/well as a feeder cell (a different cell type in a co-culture that induces proliferation of the cells).

On the third day after the plates were seeded, 50 µl/well of HAT medium was added to each of the plates. Subsequently, the additions and exchanges of the HAT medium were repeated, and the culture plates were left until the proliferating colonies of the hybridoma cells grew up to have a size 1/4-1/3 of the well diameter.

When the proliferated cell nests had grown to an appropriate size, the culture supernatant was collected, and a positive/negative test on antibody production was performed. The test was carried out by checking for immunohistochemical staining on a paraffin-embedded slice of human fetal brain of 9-14 weeks of fetal age using a Nichirei Multi Stain Kit.

A hybridoma cell isolate exhibiting antibody production was cloned through a limiting dilution method. Specifically, after the limiting dilution, the hybridoma cells were seeded on a 96 well-culture plate at about 0.5 hybridoma cells/well. The culture medium was a HT culture solution + Briclone (proliferator, Dainippon Pharmaceutical Co., Ltd.) to provide culture conditions in which only the hybridoma cells could easily survive and proliferate, so as to obtain a homogeneous cell population derived from a single hybridoma cell.

The above process between colony formation of the hybridoma cell and the cloning through the limiting dilution method was repeated 3 times. In this process, HT culture solution + Briclone was used as a culture solution. Further, only the clones having high-proliferation potential, high antibody secreting ability, and high stability at the time the ratio of antibody-producing clones to the hybridoma cells became about 1:1 were collected, and multiplied in a proliferation medium.

Hybridoma culture supernatants from 420 wells were screened by incubating them with paraffin-embedded sections of developing human brain (9-14 gestational weeks). About one fifth of the supernatants were found to be immunoreactive on the paraffin-embedded sections. The hybridomas (and antibodies produced thereby) were termed "HFB", as in **H**uman **F**etal **B**rain antigen, followed by the well number. For example, HFB115 was the hybridoma from well number 115. Similarly, HFB115 may refer to the antibody produced by hybridoma HFB 115.

### Propagation of Hybridoma Cells:

0.5 ml of pristane was injected into the abdominal cavities of mice a total of 2-3 times (once per week) for 2-3 weeks, and then 1 x 10⁷ hybridoma cells of four different hybridomas (HFB16, HFB27, HFB115 and HFB211) were separately intraperitoneally-injected into the mice (one hybridoma per mouse).

After 7-10 days from the injection of the cells, a large quantity of ascites had formed in the mice and was collected with a syringe or the like, and quickly frozen in liquid nitrogen. It was cryopreserved at -80°C.

### 2. IDENTIFICATION OF MOLECULES RECOGNIZED BY THE ANTIBODIES

### Purification and Identification of the Antigen recognized by the HFB115 antibody:

Cell aggregations (neurospheres) containing human fetal neural stem cells or neural precursor cells were produced using the method set forth in Kanemura et al., *Journal of Neuroscience Research* 69, 869-879 (2002).

Approval to use human fetal neural tissues was obtained by the ethical committees of both the Osaka National Hospital and the Tissue Engineering Research Center. Tissue procurement was in accordance with the Declaration of Helsinki and in agreement with the ethical guidelines of the Network of European CNS Transplantation and Restoration (NECTAR) and the Japan Society of Obstetrics and Gynecology.

Forebrain tissues from human fetuses aged 7 to 10 gestational weeks ("GW") were obtained from routine legal terminations performed in the Osaka National Hospital. The fetal brain tissue samples were mechanically dissected in Dulbecco's modified Eagle's Medium (DMEM)/Ham's F-12 (1:1). After dissection, the tissue samples were enzymatically digested with 0.05% trypsin/0.53 mM EDTA (Invitrogen, Carlsbad, CA, USA) for 20 min at 37°C. The trypsin activity was then stopped by adding soybean trypsin inhibitor (2.8 mg/ml, Roche Diagnostics, Mannheim, Germany). After three washes in DMEM/F-12, the tissue samples were triturated using a fire-polished Pasteur pipette, then passed through a 40-µm nylon mesh (Cell Strainer; Becton-Dickinson, Franklin Lakes, NJ, USA) to obtain single-cell suspensions. Cell numbers and viability were assessed by trypan blue dye exclusion using a hemocytometer.

Cell suspensions were grown using the neurosphere technique (Reynolds et al., Journal of Neuroscience 12, 4565-4574, 1992; Reynolds and Weiss, Developmental Biology 175, 1-13, 1996; Vescovi et al., Neuron 11, 951-966, 1993). The growth medium was a defined DMEM/F-12 (1:1)-based medium supplemented with human recombinant ("hr-") EGF (20 ng/ml; Invitrogen), hr-FGF2 (20 ng/ml; PeproTech Inc, Rocky Hill, NJ, USA), hr-LIF (10 ng/ml; Chemicon International, Inc., Temecula, CA, USA), heparin (5 µg/ml; Sigma, St. Louis, Missouri, USA), B27 supplement (Invitrogen), 15 mM HEPES, penicillin (100 units/ml; Invitrogen), streptomycin (100 µg/ml; Invitrogen), and amphotericin B (250 ng/ml; Invitrogen) (Carpenter et al, *Experimental Neurology,* 158, 265-278 (1999)); Svendsen et al., Journal of Neuroscience Methods 85, 141-152, 1998, Vescovi et al., Journal of Neurotrauma 16, 689-693, 1999, Vescovi et al., Experimental Neurology 156, 71-83, 1999).

Viable single cells at a density of 2×10⁶ cells/ml were seeded into uncoated T75 culture flasks and incubated at 37°C in 5% CO₂-95% air. Half of the volume of culture medium was replaced by fresh growth medium every 4-5 days. For passaging, every 14-21 days neurospheres were dissociated to single cells by digestion with 0.05% trypsin and 0.53 mM EDTA (Invitrogen) at 37°C for 20 min, and re-suspended in 50% fresh growth medium plus 50% neurosphere-conditioned medium.

After 6 months culture, individual cell aggregates were collected and centrifuged at 300 x g for 5 minutes to remove supernatant medium. Then, the deposited cell aggregations were rinsed with PBS (137 mM NaCl, 8.1 mM Na₂HPO₄·12H₂O, 2.68 mM KCl, 1.47 mM KH₂PO₄) three times. The cell aggregations were frozen with liquid nitrogen, and reserved stored under -80°C.

A cell aggregation reserved under -80°C was thawed on ice. To the thawed cell aggregation was added 5 ml of homogenizing buffer (HB: 0.147 mM KCl, 50 mM Tris-HCl (pH 7.5), 1 mM EDTA) containing a protease inhibitor (available from Sigma, protease inhibitor cocktail, p-8340), and homogenized (500 rpm, 5 times). The obtained solution was centrifuged at 8,000 x g for 15 minutes. The resulting supernatant was transferred to an additional centrifugation tube. To the resulting deposit was added 5 ml of HB containing the protease inhibitor again, and the deposit was homogenized (1000 rpm, 5 times). Then, the resulting solution was centrifuged at 8,000 x g for 15 minutes, and the resulting supernatant was mixed with the previously separated supernatant. A fraction of the mixed supernatant was centrifuged at 100,000 x g for 90 minutes to obtain a deposit. To the resulting deposit was added 2 ml of HB, 0.5% Triton X-100 (hereinafter referred to as HB'), and the deposit was homogenized (5000 rpm, 20 times). The obtained solution was centrifuged at 30,000 x g for 30 minutes, and the resulting supernatant was used as the cell membrane fraction.

The cell membrane fraction was subjected to column chromatography using 1 ml Hitrap Heparin HP (available from Amersham Biosciences) under the following conditions. HB' was used as equilibrium buffer, and HB', 1M NaCl was used as elution buffer. 2 ml of the cell membrane fractions were applied to heparin column, and then the column was rinsed with 2 ml of equilibrium buffer. Then, the cell membranes were eluted with 5 ml of 50% elution buffer, and the eluted fractions were separated in increments of 1 ml (Nos. A8 - A12). Then, the cells were eluted with a gradient of 50% to 100% in 15 ml of elution buffer, and the eluted fractions were separated in increments of 0.5 ml (Nos. B12 - D8).

The respective eluted fractions obtained from the heparin column chromatography were subjected to SDS polyacrylamide gel electrophoresis (SDS-PAGE) using 5%-20% polyacrylamide gradient gels (available from BioRad) according to a Laemmli method (Laemmli, U.K., *Nature,* 227, 680-685 (1970)), and then proteins in the gel were transferred onto a nitrocellulose membrane (available from Advantec) through a semidry transfer method.

In order to prevent non-specific adsorption of antibody to the membrane, the membrane with the proteins was treated with PBS plus 0.5% Tween 20 (hereinafter referred to as PBS-T) and 5% skim milk at room temperature for 1 hour. Then, to separate aliquots of the membrane was added one of three different primary antibody solutions (ascites fluid from the HFB16, HFB27 and HFB115 hybridomas) diluted by PBS-T (1000 x dilution), and an antigen-antibody reaction was permitted to occur at 4°C overnight. Then, the membranes were rinsed with PBS-T, and the proteins on the membranes were reacted with a secondary antibody at room temperature for one hour. A 1000 times-diluted solution of anti-mouse IgG-HRP or anti-mouse IgM-HRP (bridged complex of immunoglobulin G and horseradish-derived peroxidase, available from Amersham Biosciences) was used as the secondary antibody. Then, the membranes were methodically rinsed with PBS plus 0.5% Tween 20, and chemiluminescence was introduced therein through an ECL method using an ECL Plus kit (available from Amersham Biosciences). The membranes were then put into a cassette together with an X-ray film, and the X-ray film was exposed to the chemiluminescence of the membranes in a darkroom and developed.

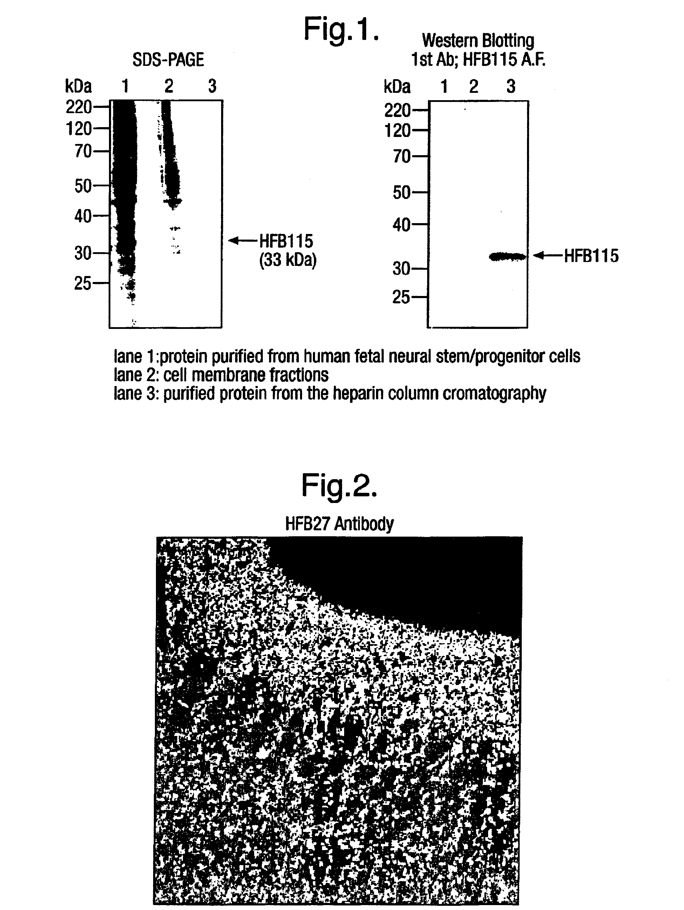

Figure 1 shows the results of the SDS-PAGE and Western blotting using the antibodies produced by HFB115. A molecule having a molecular mass of about 33 kDa was identified using the antibody, and this protein was termed the HFB115 antigen protein. The results also verified that the HFB 115 antigen protein could be purified through a purification method using a heparin column.

### Antigens recognized by the HFB16 antibody and the HFB27 antibody:

A cell aggregation (neurosphere) containing human fetal neural stem cells or neural precursor cells, cultured through the aforementioned method of Kanemura et al., was fragmented in lysis buffer containing 50 mM Tris-HCl (pH 7.5), 150 mM NaCl, 1% Nonidet P-40 (NP-40), and protease inhibitor cocktail (available from Sigma) to prepare samples (homogenates). The homogenates were subjected to SDS polyacrylamide gel electrophoresis (SDS-PAGE) using 5%-20% polyacrylamide gradient gels (available from BioRad). Then, the proteins in the gel were transferred onto a PVDF membrane (available from BioRad) through a semidry transfer method.

Western blotting proceeded as described above, using the HFB16 antibody and the HFB27 antibody. The resulting Western blot showed that the HFB27 antibody recognized protein bands of 16 kDa and 34 kDa, and the HFB16 antibody recognized a protein band of 34 kDa.

### Determination of Antibody Subclasses:

From the result of Western blotting using IgG-HRP or IgM-HRP as the secondary antibody, and a measurement using an antibody subclass assay kit (Mouse Monoclonal Antibody Isotyping Test Kit (MMT1) DAINIPPON PHARMACEUTICAL CO., LTD, Japan), it is believed that the HFB27 antibody and the HFB115 antibody belong to the IgM subclass, and the HFB16 antibody and the HFB211 antibody belong to the IgG subclass.

### 3. ANALYSIS OF INTERNAL AMINO ACID SEQUENCE OF ANTIGEN PROTEIN RECOGNIZED BY HFB 115 ANTIBODY

Column chromatography of the antigen recognized by the HFB115 antibody, and purified as described above, was performed using Hitrap Heparin HP columns (Amersham Biosciences). Fractions of the eluant from the column were collected. Fractions B2, B1, C1 and C2 containing fragments of the HFB115 antigen protein were condensed through an acetone precipitation method. To 400 µl of each of the fragment samples was added 1200 µl of acetone (-80°C), and then the samples were left at -80°C for 3 hours. This solution was then centrifuged at 20,000 x g for 15 minutes, and the obtained deposit of HFB115 antigen protein was dried. Then, the dried deposit was dissolved with 15 µl of SDS-PAGE sample buffer (Laemmli U.K., *Nature* 227, 680-685 (1970)). The solution was subjected to SDS-PAGE using 12% discontinuous polyacrylamide gel, and then the gel was stained with Coomassie G-250 and adequately decolorized to identify and isolate a HFB115 antigen protein band of 33 kDa. The isolated HFB115 antigen protein band was kept at -20°C. After purification using SDS-PAGE, HFB115 antigen protein band of 33kDa was digested using trypsin. The internal amino acid sequence of the 33 kDa HFB115 antigen protein band was determined from the digestion (by APRP Life Science Institute, Inc.).

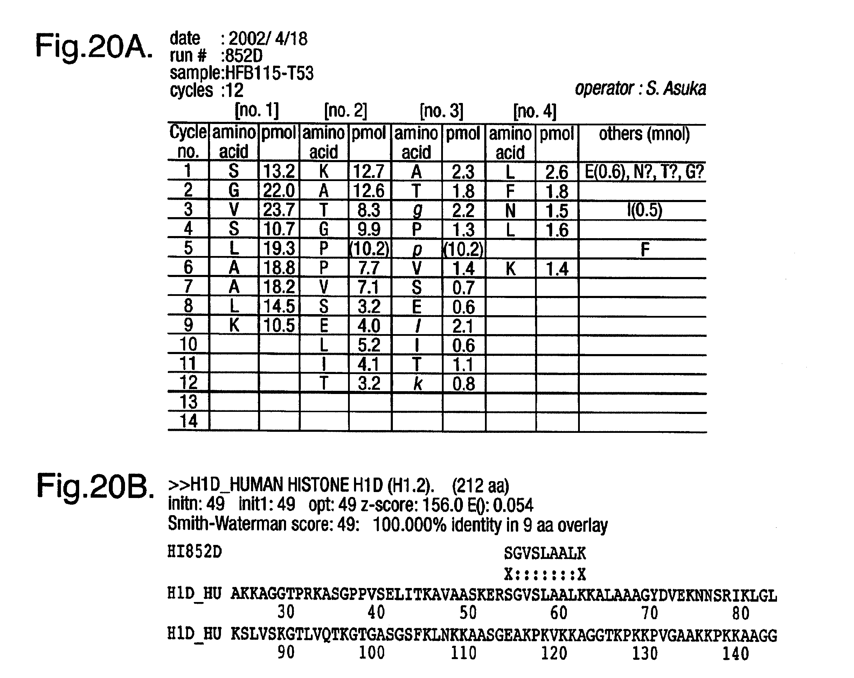

Figure 20 shows the results of the analysis of the internal amino acid sequence of the HFB 115 antigen protein. The analysis results indicated that from three different peptide fragments were produced by the digestion, and the thirteen longest amino acid sequences revealed that the HFB 115 antigen protein had 100% homology to the human histone H1D (H1.2).

### 4. COMPARISON BETWEEN RECOGNITION REGIONS OF THE HFB115

### Antibody and An Anti-Histone H1 Antibody:

A frozen section of a cell aggregation (neurosphere) containing human fetal neural stem cells or neural precursor cells, cultured through the aforementioned method of Kanemura et al., was prepared. The section was rinsed with PBS, and subjected to blocking treatment using PBS containing 10% goat serum and 0.01% Triton X-100, at room temperature for 1 hour. Then, the section was reacted with the HFB115 antibody (500 times dilution), an anti-Histone H1 monoclonal antibody (500 times dilution), and an anti-Histone H1 polyclonal antibody (500 times dilution), the latter two antibodies being available from Santa Cruz Biotechnology, at 4°C for one night. After completion of the reaction, the section was rinsed with PBS, and reacted with a secondary antibody mixture (Alexsa anti-mouse 1gM antibody 647, Alexsa anti-mouse IgG antibody 488, Alexsa anti-rabbit IgG antibody 568, each available from Molecular Probe) at room temperature for 1 hour. The section was rinsed with PBS, ddH₂O, and then mounted. The sample was observed with a confocal laser scan microscope (LSM 510, available from Carl Zeiss).

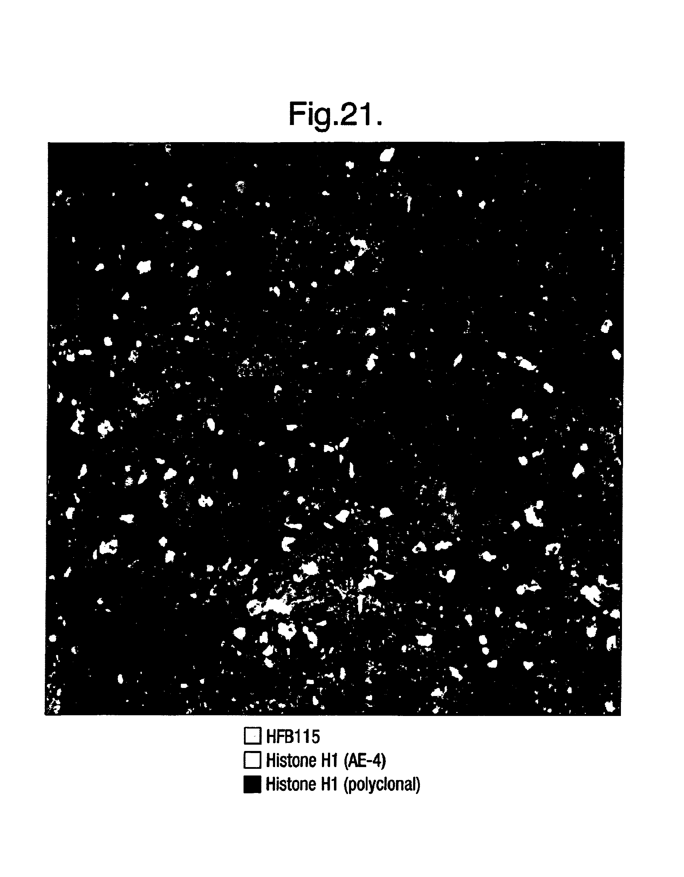

The comparative test using frozen sections of neurospheres showed that the HFB115 antibody had a different recognition region from that of the anti-Histone H1 antibodies (Figure 21). While anti-Histone H1 antibody recognizes nucleus (anti-Histone H1 monoclonal antibody) or cytoplasm (anti-Histone H1 polyclonal antibody), the HFB115 antibody recognizes cell membrane, or intercellular substance (or matrix).

### Immunostaining of Human Fetal Brain Using Antibody:

To determine the immunostaining pattern of Human fetal brain using the antibodies of the present invention, a tissue slice cut out from a paraffin-embedded block was treated 3 times with histoclear, and then treated with 100%, 95%, 90%, 80%, and 70% ethanol solutions in this order for 5 minutes each. As a blocking treatment for suppressing the non-specific absorption of antibody, the slices were rinsed with PBS three times for 5 minutes each, and then rinsed with a blocking solution (PBS containing 10% intact goat serum and 0.01% Triton X-100) for 1 hour. The antibody diluted to 0.02% with PBS containing 10% intact goat serum and 0.01% Triton X-100 was reacted with this tissue slice sample at 4°C for 15 hours. The resulting sample was rinsed with PBS five times for 5 minutes each, and then reacted with the secondary antibody solution, which contains an anti-mouse immunoglobulin-FITC complex diluted to about 0.02% with the blocking solution, at room temperature for 1 hour. After rinsing with PBS, five times for 5 minutes each, the resulting sample was observed with a fluorescence microscope to check stained regions.

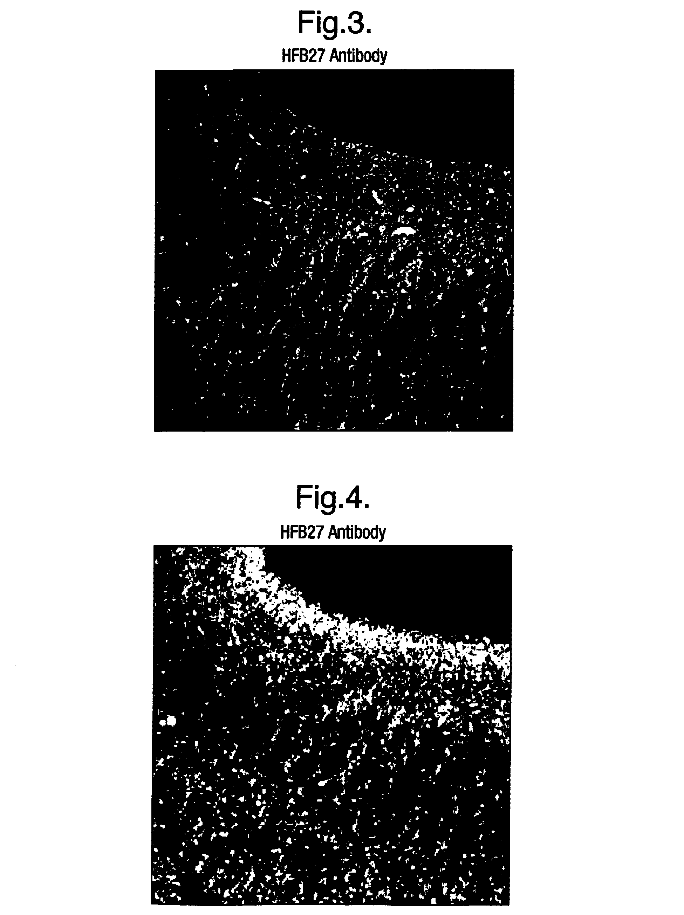

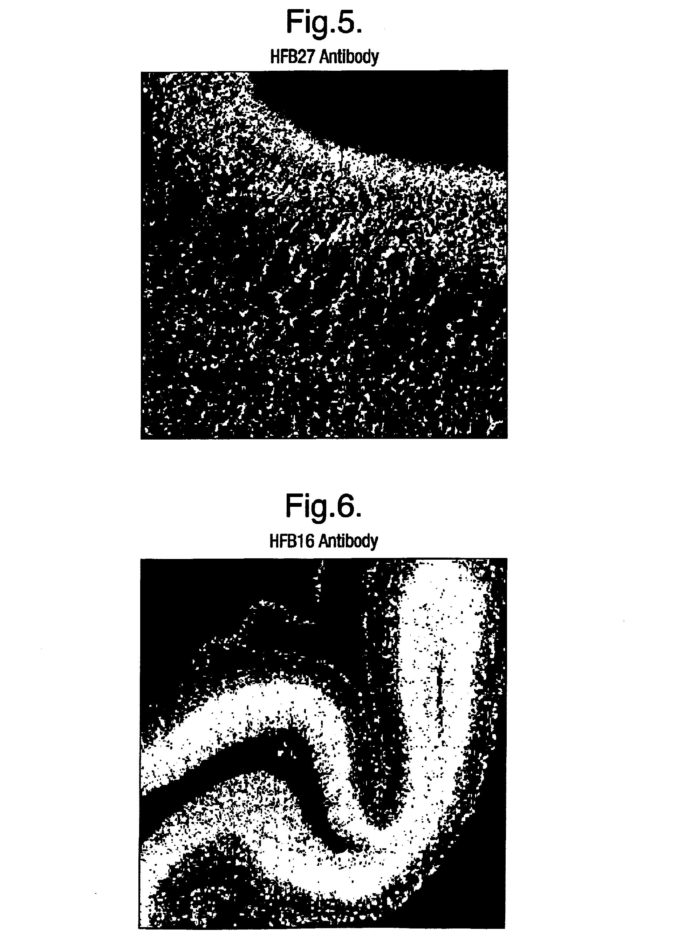

Figs. 2 to 5 show immunostain images of human fetal brain tissue using the HFB27 antibody. Among them, Fig. 2 is an image showing a nuclear stain using the antibody with DNA staining dye TO-PRO-3®, or the location of the cells. Fig 3 is an immunostain image resulting from the antibody in Nestin which is a known marker protein expressed on neural stem cells or precursor cells. Fig. 4 is an image of immunostain resulting from the HFB27 antibody. Fig. 5 is a superimposed multiple stain image of the above three stain images. The HFB27 antibody selectively stains a region of the ependymal and subependymal layers around brain ventricle.

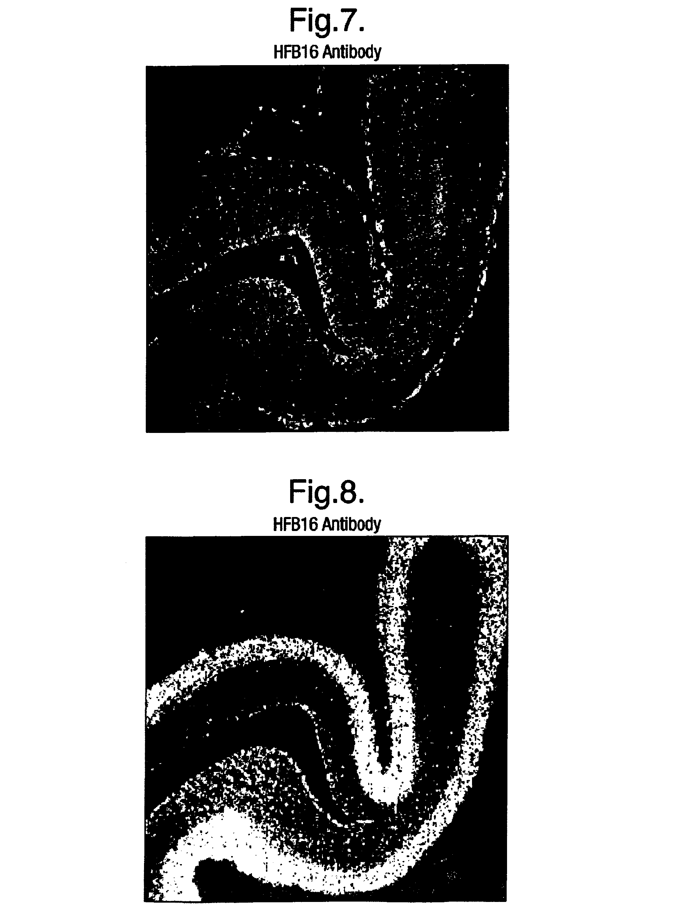

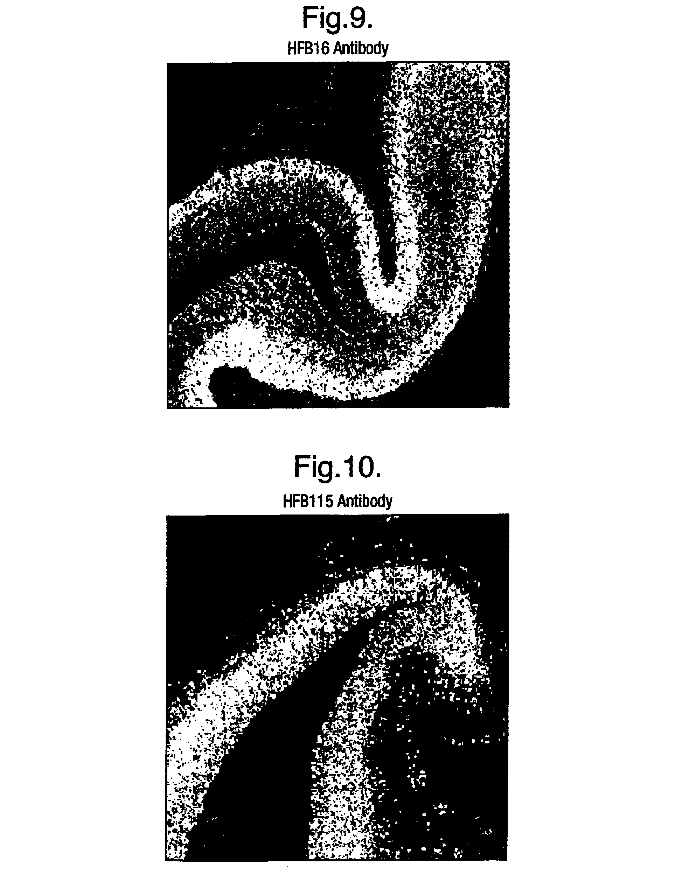

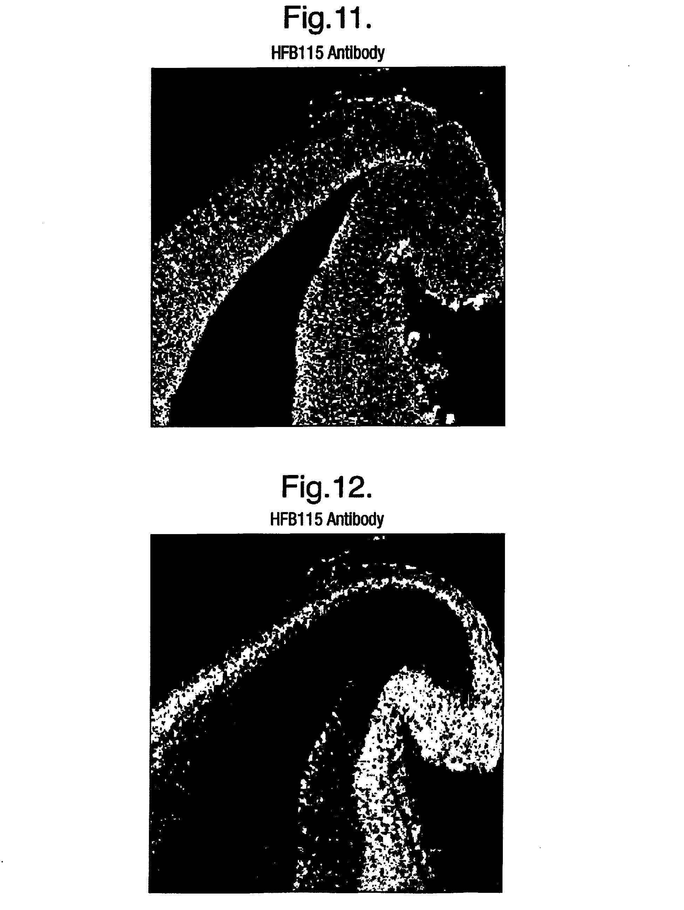

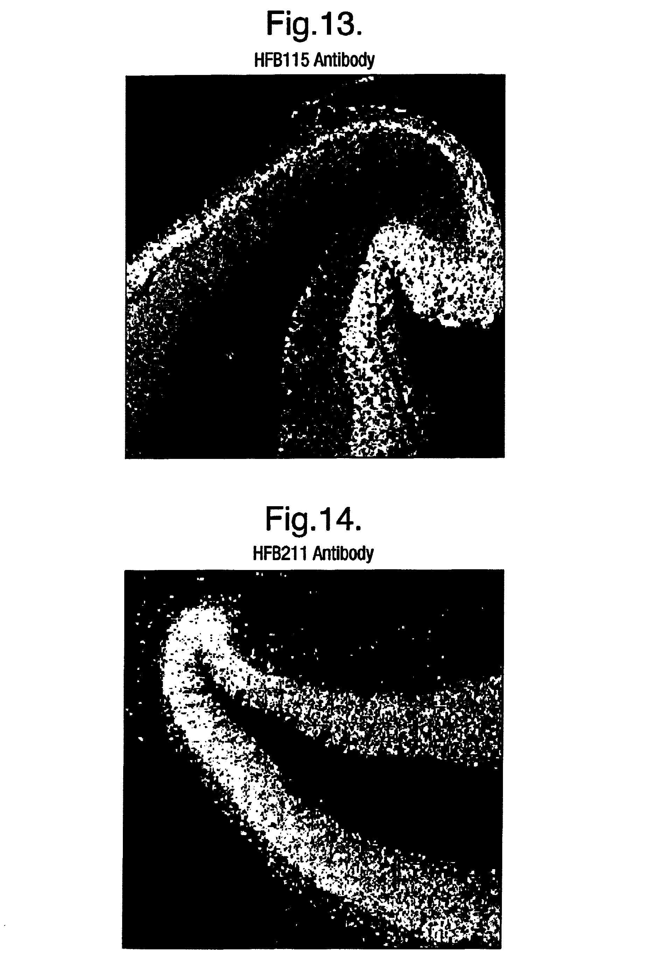

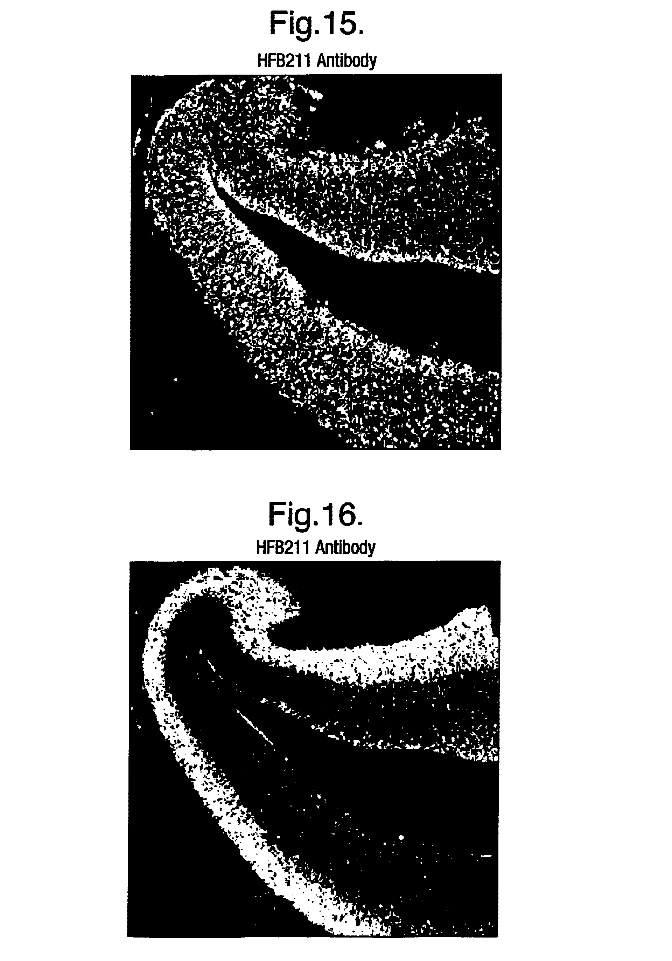

In the same order, Figs. 6 to 9 show immunostain images resulting from HFB16 antibody, Figs. 10 to 13 showing immunostain images resulting from HFB115 antibody, and Figs. 14 to 17 showing immunostain images resulting from HFB211 antibody. Based on the results from the use of the HFB16 antibody and the HFB211 antibody, a small part of the ependymal layer around brain ventricle wall is stained and the subependymal layer has almost no stain, while a remaining region other than the ependymal and subependymal layers is stained. Similarly, based on the results from the use of the HFB115 antibody it can be seen that the ependymal and subependymal layers have almost no stain, and the remaining regions far from the brain ventricle are stained.

As seen in these results, the HFB27 antibody is a monoclonal antibody capable of selectively staining the ependymal and subependymal layers of brain tissue in the immunostaining test using human brain tissue slices. The HFB16 antibody, the HFB115 antibody and the HFB211 antibody are monoclonal antibodies capable of selectively staining a region other than the ependymal and subependymal layers of human brain tissues, and do not stain the ependymal and subependymal layers. The above four kinds of monoclonal antibodies can be used in immunohistochemical diagnostic methods of brain tissue slices.

### Cell Labeling and FACS:

Constitutive cells, neurospheres containing human fetal-derived neural stem cells or nervous system precursor cells, were physically separated by repeatedly entering in and exiting a Pasteur pipette, and about 300 g of the separated cells were subjected to centrifugation for about 5 minutes to collect the cells. Separate aliquots of the obtained cells were treated at 4°C for 30 minutes with medium containing 0.8% primary antibody from each of the four hybridomas (HFB16, HFB27, HFB115 and HFB211) and 17% goat IgG, and then rinsed in a medium without the primary antibody, three times for 5 minutes each. A secondary antibody solution (1% anti-mouse IgG-FITC complex or anti-mouse IgM-FITC complex) was added to and reacted with the cells at 4°C for 25 minutes in a dark box. After rinsing three times for 5 minutes each in a medium without the secondary antibody, the cells were suspended in a medium containing 1 µg/ml 7-amino-actinomycin D (VIA-PROBE, Becton Dickinson) to form a sample for FACS analysis, and sorted using a cell sorter (FACS Vantage SE, Becton Dickinson). When Jurkat cells were used in control experiments, Propidium Iodide (PI) was used as substitute for VIA-PROBE.

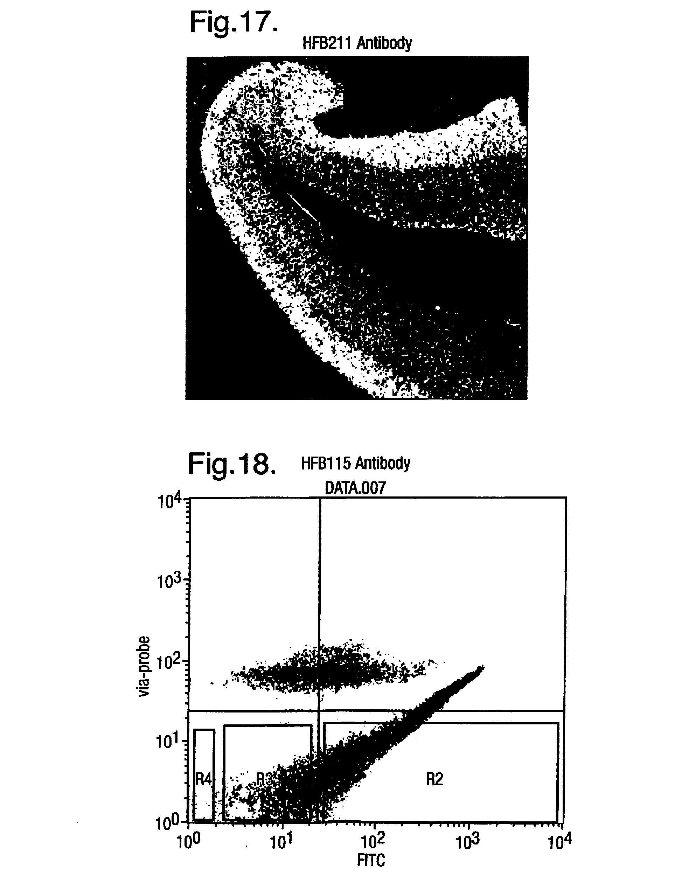

Fig. 18 shows the sorting result of neurosphere cells using the HFB115 antibody. The horizontal axis indicates fluorescence intensity caused by the secondary antigen FITC, which corresponds to the amount of the antibody linked to the cell surfaces. The vertical axis indicates fluorescence intensity caused by the dye selectively staining dead cells, wherein the high fluorescence intensity value means a high possibility that the stained cell is dead. The cell populations in Fig. 18 were separated into two clusters. The horizontally extending cell population in the center of the vertical axis and in the relatively left side of the horizontal axis would contain a number of dead cells. The cell population extending from the lower left region to the center of the right region in Fig. 18 must be living cells having antigens on the outer surfaces of the cells. From the above analysis, it is clear that the HFB115 antibody recognizes the antigens on the surface of the cells because many cells were observed in R2 frame in the sorting using the HFB 115 antibody. Similar sorting results were obtained for the HFB 16 antibody and the HFB211 antibody.

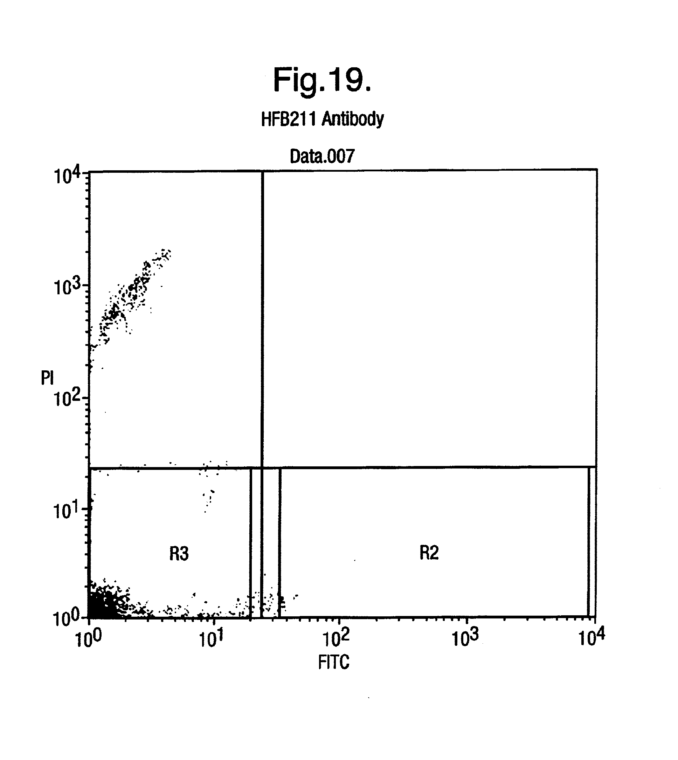

Fig. 19 shows an example of a sorting result of the Jurkat lymphoid cell line (a human leukemic T cell line). As shown in this example, the HFB115 antibody and the HFB211 antibody can also be used to isolate cells other than nerve cells through a sorting method or the like, as long as the cells express the same antigen.

The hybridomas which produces the monoclonal antibodies of the present invention are a novel fused cells, and each has been deposited in an official microorganism depositary organization of International Patent Organism Depositary, National Institute of Advanced Industrial Science and Technology, Japan, AIST Tsukuba Central 6, 1-1-1 Higashi, Tsukuba, Ibaraki, Japan on March 12, 2002. The deposit numbers are as follows:
Hybridoma HFB16: FERM P-18778
Hybridoma HFB27: FERM P-18779
Hybridoma HFB115: FERMP-18780
Hybridoma HFB211: FERM P-18781

As described above, the monoclonal antibodies of the present invention can selectively recognize cells other than a neural stem cell or a neural precursor cell among neural cells, and be used to efficiently isolate neural stem cells or precursor cells from human brain tissue. Further, the monoclonal antibody of the present invention can selectively recognize cells other than neural stem cells or precursor cells and thus prevent fluorescent materials from depositing on the target stem or precursor cell so as to assure the safety of the isolated stem or precursor cell. Furthermore, by isolating such safe neural stem or precursor cells, and controlling the proliferation/differentiation of the isolated neural stem or precursor cells, the monoclonal antibody of the present invention can be applied to medical diagnosis or regeneration treatments for neurological disorders.

## Claims

1. A monoclonal antibody which selectively recognizes an antigen expressed on cells localized in the ependymal layer and subependymal layer of the human brain.

2. A monoclonal antibody which selectively stains the ependymal layer and subependymal layer of human brain tissue in an immunostaining procedure perform on a human brain tissue sample.

3. A monoclonal antibody which recognizes an antigen expressed by cells in a region of the human brain other than the ependymal layer and subependymal layer of the human brain, and that does not recognize an antigen expressed by cells in the ependymal layer and subependymal layer of the human brain.

4. A monoclonal antibody which selectively stains a region of the human brain other than the ependymal layer and subependymal layer of the human brain in an immunostaining procedure using a human brain tissue sample.

5. A hybridoma which produces a monoclonal antibody as defined in any one of claims 1 to 4.

6. A neural cell isolation method, said method comprising contacting a cellular sample with an antibody as defined in any one of claims 1 to 4, and isolating cells marked with said antibody from said sample.

7. A neural cell which is isolated through the neural cell isolation method as defined in claim 6.

8. A cell isolation method, said method comprising contacting a cellular sample with an antibody as defined in any one of claims 1 to 4, and isolating cells marked with said antibody from said sample.

9. An immunological diagnostic method utilizing an antigen-antibody reaction caused by the monoclonal antibody as defined in any one of claims 1 to 4, said method comprising preparing a biological sample, contacting said biological sample with one or more of said monoclonal antibodies, and detecting antibody binding, wherein binding demonstrates the presence of a marker associated with a disease or disorder of the central nervous system.

10. A hybridoma cell line HFB16 deposited with the International Patent Organism Depositary, National Institute of Advanced Industrial Science and Technology, Japan as accession number FERM P-18778.

11. A monoclonal antibody HFB16, or antigen-binding fragment thereof, which is produced by the hybridoma cell line HFB16 deposited with the International Patent Organism Depositary, National Institute of Advanced Industrial Science and Technology, Japan as accession number FERM P-18778.

12. A hybridoma cell line HFB27 deposited with the International Patent Organism Depositary, National Institute of Advanced Industrial Science and Technology, Japan as accession number FERM P-18779.

13. A monoclonal antibody HFB27, or antigen-binding fragment thereof, which is produced by the hybridoma cell line HFB27 deposited with the International Patent Organism Depositary, National Institute of Advanced Industrial Science and Technology, Japan as accession number FERM P-18779.

14. A hybridoma cell line HFB115 deposited with the International Patent Organism Depositary, National Institute of Advanced Industrial Science and Technology, Japan as accession number FERM P-18780.

15. A monoclonal antibody HFB 115, or antigen-binding fragment thereof, which is produced by the hybridoma cell line HFB115 deposited with the International Patent Organism Depositary, National Institute of Advanced Industrial Science and Technology, Japan as accession number FERM P-18780.

16. A hybridoma cell line HFB211 deposited with the International Patent Organism Depositary, National Institute of Advanced Industrial Science and Technology, Japan as accession number FERM P-18781.

17. A monoclonal antibody HFB211, or antigen-binding fragment thereof, which is produced by the hybridoma cell line HFB211 deposited with the International Patent Organism Depositary, National Institute of Advanced Industrial Science and Technology, Japan as accession number FERM P-18781.

18. The monoclonal antibody as defined in any one of claims 1 to 4, wherein said antibody specifically recognizes Histone H1.

19. A hybridoma which produces a monoclonal antibody that specifically recognizes Histone H1.

20. The monoclonal antibody as defined in any one of claims 1 to 4, wherein said antibody specifically recognizes cell-surface Histone H1, but does not recognize intranuclear Histone H1.
